# EUROPEAN PATENT SPECIFICATION

(11) **EP 2 440 508 B1**
(45) Date of publication and mention of the grant of the patent: **21.05.2014**
(21) Application number: 10728287.3
(22) Date of filing: 02.06.2010
(51) Int. Cl.: C07C 2/86, C07C 15/46

(54) **PROCESS FOR THE ALKYLATION OF BENZENE WITH ETHANOL OR A MIXTURE OF ETHANOL AND ETHYLENE**
VERFAHREN ZUR ALKYLIERUNG VON BENZOL MIT ETHANOL ODER EINER MISCHUNG AUS ETHANOL UND ETHYLEN
PROCÉDÉ D'ALKYLATION DU BENZÈNE AVEC DE L'ÉTHANOL OU UN MÉLANGE D'ÉTHANOL ET D'ÉTHYLÈNE

(30) Priority: 10.06.2009 IT MI20091022
(43) Date of publication of application: 18.04.2012
(73) Proprietor: versalis S.p.A., 20097 San Donato Milanese (MI) (IT)
(72) Inventor: LORENZONI, Loreno, Loredano, 07046 Porto Torres (SS) (IT); BENCINI, Elena, I-46030 Virgilio (MN) (IT); CASALINI, Alessandro, I-46100 Mantova (IT)
(74) Representative: Bottero, Carlo
(86) International application number: PCT/IB2010/001349
(87) International publication number: WO 2010/143043

(56) References cited:
- EP-A1- 0 012 514
- EP-A1- 1 069 100
- US-A- 4 387 259
- US-A- 4 469 908
- CHANDAWAR ET AL: "ALKYLATION OF BENZENE WITH ETHANOL OVER ZSM5 ZEOLITES" APPLIED CATALYSIS, vol. 4, 1 January 1982 (1982-01-01), pages 287-295, XP002573251 AMSTERDAM ISSN: 0166-9834

## Description

The present invention relates to a process for the alkylation of benzene with ethanol, as alkylating agent, or mixtures of ethanol and ethylene, comprising effecting said reaction in gaseous phase or mixed gas-liquid phase, and in the presence of a catalytic system containing a zeolites belonging to the MTW family.

According to a preferred aspect, ethanol obtained from biomasses is used, in particular from the fermentation of sugars deriving from biomasses.

The process of the present invention is characterized by the absence of negative effects on performances and duration of the catalyst due to the presence of high quantities of water in the reaction mixture as well as by-products deriving from undesired reactions, and also provides much higher productivities.

The absence of negative effects is due to the particular catalytic system used which proves to be particularly suitable for the alkylation of benzene with ethanol, or mixtures of ethanol and ethylene, as alkylating agent, under the reaction conditions selected.

The invention also relates to a process for preparing styrene in which the first preparation step of ethylbenzene is effected by the alkylation of benzene according to what is specified above.

Ethylbenzene is an important intermediate product of basic chemical industries, mainly used as precursor for the production of styrene, in turn useful as intermediate in the preparation of styrene polymers and copolymers. The industrial synthesis of styrene comprises the alkylation steps of benzene to ethylbenzene and the transformation of ethylbenzene into styrene by a dehydrogenation reaction.

For the alkylation of benzene with ethylene to give ethylbenzene in addition to zeolitic catalysts, AlCl₃ is still partly used in the petrochemical industry, as catalyst, in a slurry reactor. Problems of environmental impact and safety are linked to processes based on the use of AlCl₃: the use of this catalyst, in fact, is particularly problematical due to the corrosion and disposal of the exhausted catalyst.

The use of zeolites with a faujasite structure for the alkylation of benzene with light olefins such as ethylene and propylene is described by Venuto et al. (J.Catal.5, (1966) 81).

Optimum results, in terms of industrial application, have been obtained in the synthesis of ethylbenzene starting from benzene and ethylene using zeolites with a beta-type structure, as described in EP 432814, and in particular using catalysts comprising zeolite beta according to what is described in EP 687500.

The direct use of ethanol in the alkylation of benzene to give ethylbenzene with acid catalysts of the conventional type, on the other hand, has not proved to be industrially practicable due to the water released from the ethanol during the reaction which produces negative effects on the performances of the catalyst in terms of selectivity, but above all duration of the catalyst itself.

Acid catalysts of both the zeolite and non-zeolite type are in fact negatively influenced by the presence of water which is developed when ethyl alcohol is used as alkylating agent of benzene to give ethylbenzene.

In the case of a catalyst of the conventional type such as aluminium trichloride, used in the industrial synthesis of ethylbenzene, quantities of water exceeding a few hundreds of ppm in the reaction mixture produce a considerable reduction in the catalytic performances in terms of yield to ethylbenzene.

In the case of zeolite-based catalysts, the negative effect is known, due to the presence of water, which is revealed with a lowering of the overall yield to ethylbenzene combined with a more or less rapid deactivation of the catalyst itself.

All of these negative effects are known and also verified with extremely low contents of water - present in the reaction - with respect to those obtained using ethyl alcohol as alkylating agent of benzene to give ethylbenzene in a process of concrete industrial applicability.

The industrial applicability of an alkylation process of benzene with ethyl alcohol, in fact, cannot disregard certain parameters such as, for example, the benzene/ethanol molar ratio in the feed to the reaction section, which generally ranges from 3 to 10 with a corresponding concentration of water in the reaction equal to about 64,000 and 21,000 ppm, assuming the total conversion of the ethyl alcohol.

Even effecting the alkylation of benzene with an alkylating agent consisting of a mixture of ethanol and ethylene, it would in any case be necessary to significantly reduce the quantity of ethyl alcohol used to guarantee a water content which could be tolerated by the catalytic system, thus limiting the actual potentiality of the process itself.

C.J. Johney, A.J. Chandwadkar, G.V. Potnis, M.U.Pai, S.B. Kulcarni, in "Indian Journal of Technology", vol.15, November 1977, pages 486-489, describe the alkylation of benzene with ethanol, at atmospheric pressure, in the presence of 13-X zeolites variably substituted. The activity of these catalysts is not very high and rapidly decreases.

K.H. Chandawar, S.B. Kulkarni, P. Ratnasamy, in "Applied Catalysis", 4(1982), 287-295, describe the alkylation of benzene with ethanol in the presence of ZSM-5 zeolite, acceptable conversions however are only obtained by operating at very high temperatures. The catalysts used for the alkylation of benzene with ethylene are consequently not easily transferable to the alkylation reaction of benzene with ethyl alcohol, or mixtures of ethyl alcohol and ethylene, as alkylating agent, as these catalysts are generally extremely sensitive to water and their life in the presence of the water formed in the reaction is consequently extremely reduced. EP 0012514 discloses a process for the alkylation of benzene with methanol using a MTW zeolite. The process leads to a large proportion of polyalkylated products.

Ethanol, on the other hand, can be obtained from biomasses, in particular from the fermentation of sugars deriving from biomasses, and therefore represents a potential raw material for the petrochemical industry alternative to fossil sources. It is consequently strategically important and also economically relevant to define new upgradings of this product in the production of intermediates of industrial interest.

Biomass is any substance having an organic, vegetable or animal matrix, which can be destined for energy purposes, for example, as raw material for the production of biofuels, or components which can be added to fuels.

In particular, lignocellulosic biomass is a complex structure comprising three main components: cellulose, hemicellulose, and lignin. Their relative quantities vary according to the type of lignocellulosic biomass used. Cellulose is the greatest constituent of lignocellulosic biomass and consists of glucose molecules (from about 500 to 10,000 units) bound to each other through a β-1,4 glucoside bond. Hemicellulose, which is generally present in a quantity ranging from 10% by weight to 40% by weight with respect to the total weight of the lignocellulosic biomass appears as a mixed polymer, relatively short and branched, made up of both sugars with six carbon atoms and also sugars with five carbon atoms. Lignin is generally present in a quantity ranging from 10% by weight to 30% by weight with respect to the total weight of the lignocellulosic biomass.

The synthesis of ethanol from biomass is divided into various steps and comprises the conversion of the biomass into a feed which can be used for the fermentation (normally in the form of sugars) by applying one of the many technological processes available: said conversion forms the step which differentiates the various technological solutions in the synthesis of bio-ethanol. This step is followed by the fermentation of the intermediates of the biomass using bio-catalysts (micro-organisms such as yeast and bacteria) to obtain ethanol in a low-concentration solution. The fermentation product is then processed to obtain ethanol and by-products which can be used in the production of other fuels, chemical compounds, heat and electric energy.

In the first step, in order to optimize the transformation of the lignocellulosic biomass into products for energy use, subjecting said biomass to a treatment which separates the lignin and hydrolyzes the cellulose and hemicellulose to simple sugars such as, for example, glucose and xylose, which can then be subjected to fermentation processes to produce alcohols, is known.

Various processes can be used for this purpose, in particular hydrolysis, preferably acid, which is carried out in the presence of strong mineral acids, generally H₂SO₄, HCl or HNO₃, diluted or concentrated, or enzymatic hydrolysis.

The product obtained is then subjected to fermentation for the production of alcohols, in particular ethanol.

Processes for the production of ethanol from biomasses are described for example in US 5562777 ; US 2008/0044877; "Ethanol from ligninocellulosic biomass: technology, economics and process for the production of ethanol" F. Magalhaes, R.M. Vila Cha-Baptista, 4th International Conference on Hands-on Science Development, Diversity and Inclusion in Science Education 2007; "Ethanol fermentation from biomass resources: current state and prospects" Y. Lin, S.Tanaka, Appl. Microbiol. Biotechnol. (2006) 69:627-642; hydrolysis of ligninocellulosic materials for ethanol production:a review" Y. Sun, J. Cheng, Bioresource Tecnology, volume 83, Issue 1, May 2002, pages 1-11.

We have now found that it is possible to obtain ethylbenzene by the alkylation of benzene with ethanol, as alkylating agent, or mixtures of ethanol and ethylene, by means of a process which gives better results in terms of performances, duration of the catalyst and therefore productivity, even in the presence of considerable quantities of water, operating under suitable reaction conditions and using a catalyst comprising a zeolite of the MTW type.

According to a preferred aspect, ethanol obtained from biomasses is used, in particular from the fermentation of sugars deriving from biomasses.

An object of the present invention therefore relates to a process for the alkylation of benzene with ethanol, or a mixture of ethanol and ethylene, which comprises effecting said alkylation reaction under gaseous phase or mixed gas-liquid phase conditions, and in the presence of a catalytic system containing a zeolite belonging to the MTW family.

According to an aspect of the present invention, it can be selected to operate under pressure and temperature conditions which correspond to the complete gas phase of the whole mixture present in the reaction section: in this case therefore both the reagents and the products are in gas phase. According to another aspect of the present invention, it can be selected to operate under temperature and pressure conditions which correspond to at least partial liquid phase of the reaction products: in this case therefore the reagents are in gas phase, whereas the products are, at least partially, liquid.

According to a further aspect of the present invention, it is possible to operate under temperature and pressure conditions which are such as to have reagents and products which are both in gas phase and liquid phase.

The process according to the present invention allows to operate at relatively low molar ratios between benzene and ethyl alcohol to be used in the feed to the reaction section, within a range of concrete industrial applicability, and therefore regardless of the total quantity of water developed during the reaction.

According to a preferred aspect of the present invention, ethanol obtained from the fermentation of sugars deriving from biomasses, is used.

A preferred aspect of the present invention therefore relates to a process for the alkylation of benzene with ethanol, or a mixture of ethanol and ethylene, which comprises effecting said alkylation reaction under gaseous phase or mixed gas-liquid phase conditions, and in the presence of a catalytic system containing a zeolite belonging to the MTW family, wherein ethanol is obtained from biomasses, preferably lignocellulosic biomasses.

Any of the known methods for obtaining ethanol from biomasses is suitable for providing ethanol which can be used in the present invention.

In particular, ethanol obtained from the fermentation of sugars deriving from biomasses, preferably lignocellulosic biomasses, is used, according to any of the methods known to experts in the field. Even more specifically, the ethanol used, is obtained with a process in which the biomass, preferably lignocellulosic, is transformed into a feed which can be adopted for fermentation, preferably in the form of sugars, and then subjected to fermentation.

A particular object of the present invention therefore relates to a process for the alkylation of benzene with ethanol, or a mixture of ethanol and ethylene, comprising:
1) subjecting the biomass, preferably a lignocellulosic biomass, to transformation to obtain a feed which can be used for fermentation, said feed preferably being in the form of sugars,
2) subjecting the feed thus obtained to fermentation to obtain ethanol,
3) alkylating benzene with the ethanol thus obtained, possibly mixed with ethylene, under gaseous phase or mixed gas-liquid phase conditions, and in the presence of a catalytic system containing a zeolite belonging to the MTW family.

The first step can be effected by treatment with an acid or by enzymatic hydrolysis (SHF process). According to a particular aspect, the first and second step can be effected simultaneously, for example in the presence of the fungus T. reesei and yeast S. cerevisiae (SSF process).

The ethanol obtained from step (2) is separated, for example by means of distillation.

In the process of the present invention, zeolites of the MTW structural type which can be used are, for example, ZSM-12, CZH-5, Nu-13, Theta-3 and TPZ-12. The zeolite CZH-5 is described in GB 2079735A; Nu-1 is described in EP59059; Theta-3 is described in EP 162719 and TPZ-12 in US 4,557,919. The zeolite of the MTW structural type preferably used is a silico-aluminate with an SiO₂ /Al₂O₃ molar ratio higher than or equal to 20. This zeolite is described in A Katovic and G.Giordano, Chem. Ind. (Dekker) (Synthesis of Porous Materials) 1997 69, 127-137. The aluminium can be totally or partially substituted by B, Ga, Fe or mixtures thereof, as described by Toktarev & Ione, in Chon et al., Progress in Zeolites and Microporous Material, SSSC, vol.105, 1997. According to a preferred aspect of the present patent application, ZSM-12 zeolite is used, a porous crystalline material having in its calcined and anhydrous form a molar composition of the oxides corresponding to the following formula:

**1.0 ± 0.4 M_{2/n} O. W₂ O₃. 20-500 YO₂ . zH₂O**

wherein M is H⁺ and/or a cation of an alkaline or alkaline earth metal having a valence n; W is selected from aluminium, gallium or mixtures thereof, Y is selected from silicon and germanium, z ranges from 0 to 60. M is preferably selected from sodium, potassium, hydrogen or mixtures thereof. W is preferably aluminium and Y is preferably silicon. W can be at least partially substituted by boron, iron or mixtures thereof. ZSM-12 zeolite is described in US 3832449, in Ernst et al., Zeolites, 1987, Vol.7, September, and in Toktarev & Ione, Chon et al., Progress in Zeolites and Microporous Material, SSSC, Vol.105,1997.

The MTW zeolite, and in particular ZSM-12 zeolite, is preferably used in the form in which the cationic sites present in its structure are occupied for at least 50% by hydrogen ions. It is especially preferred for at least 90% of the cationic sites to be occupied by hydrogen ions.

According to an aspect of the invention, phosphorous can be added to the MTW zeolite. The addition can be effected by treatment of the zeolite, preferably in ammonia form, with a compound of phosphorous using any of the known techniques, such as mechanical mixing, impregnation or deposition in vapour phase. The phosphorous compound can be selected from the corresponding salts, acids and organic compounds, such as for example alkoxides. The impregnation technique is preferably used, i.e. the zeolite is preferably treated in ammonia form, with an aqueous solution of a compound of P. The resulting suspension, after being kept under stirring, is dried under vacuum at a temperature which is sufficient for eliminating the solvent. The modes and conditions for effecting the impregnation are known to experts in the field. The solid resulting from the drying is then calcined at a temperature ranging from 400 to 600°C for 1-10 hours. The P is preferably present in a quantity of less than 3% with respect to the total weight of the catalytic composition, and preferably in a quantity higher than or equal to 0.05% and lower than or equal to 2% by weight with respect to the total weight of the catalytic composition.

In the process of the present invention, the zeolite can be used as such or in the form bound with an inorganic ligand. It can be used in the form of pellets obtained by extrusion, for example, or in the form of microspheres obtained by means of spray-drying, these techniques also being applied to the zeolite as such or mixed with a suitable inorganic ligand. The ligand can be alumina, silica, a silico-aluminate, titania, zirconia or clay. The ligand is preferably alumina. In the bound catalyst, the zeolite and ligand can be in a weight ratio ranging from 5/95 to 95/5, preferably from 20/80 to 80/20. In a preferred embodiment, the end-catalyst is also characterized by particular characteristics of extrazeolite porosity, i.e. the porosity fraction of the catalyst which cannot be attributed to the quality and quantity of zeolite present in the end-catalyst. In particular, said extrazeolite porosity has values not lower than 0.4 ml/g of end-catalyst associated with a fraction equal to at least 50% of said extrazeolite porosity characterized by pores having a diameter greater than 100 Angstrom. Said extrazeolite porosity can be obtained with conventional preparation methods and is correctly determined according to known methods described for example in Loweel, Seymour "Introduction to powder surface area", Wiley Interscience.

According to a preferred aspect of the process of the present invention, the operating reaction temperature ranges from 200°C to 400°C, with a reaction pressure ranging from 1 to 20 bar and indifferently using ethanol or mixtures of ethanol and ethylene as alkylating agent. It is preferable to operate at a pressure lower than 10 bar, preferably between 1 and 6 bar.

In the processed claimed herein, the molar ratio between benzene and ethanol preferably varies from 2 to 20, even more preferably from 4 to 10.

When ethylene is also used additionally as alkylating agent together with ethanol, the molar ratio between benzene and alkylating agent -ethanol plus ethylene - preferably ranges from 2 to 20, more preferably from 4 to 10. The molar ratio between ethanol and ethylene preferably varies from 10 to 0.01 and even more preferably from 5 to 0.1.

The alkylation of benzene with ethanol can be effected in continuous, semi-continuous or batchwise.

When the process is carried out in continuous, it is also possible to operate using a configuration of the reaction system which includes the partial recycling to the reaction section of the organic phase of the effluent leaving the same reaction section, after cooling, demixing and removal of the aqueous phase from the organic phase.

The alkylation reaction of benzene with ethanol or mixtures of ethanol and ethylene as alkylating agent, in any case remains exothermic in spite of the presence of ethanol and in order to maintain the temperature within a preferred range and reduce the by-production of polyalkylated aromatic compounds, the catalyst can be arranged in the reactor in various layers inside a fixed bed reactor.

A quench is effected between one layer and another with inert solvents and part of the benzene and/or part of the alkylating agent, ethyl alcohol or mixture of ethyl alcohol/ethylene.

By thus operating, it is possible to obtain high benzene/alkylating agent ratios on the single layer, without increasing the same overall ratio, with an evident advantage with respect to the selectivity to ethylbenzene and consequently the separation operations downstream of the reaction section.

The temperature control can be effected not only by carrying out a quench of the reagents and/or inert products, but also by inter-cooling between the layers.

The alkylation reaction can be conveniently carried out in two or more reactors in series, inter-cooled to control the temperature. The feeding of the ethyl alcohol, possibly mixed with ethylene, and/or benzene can be suitably divided between the various reactors and different layers of the reactors, i.e, the alkylating agent and the benzene are added in more than one step.

According to a preferred aspect, in order to maximize the production of ethylbenzene, the resulting product can be separated into a fraction (1) containing benzene, a fraction (2) containing ethylbenzene and a fraction (3) containing polyethylbenzenes. The fraction (1) can be re-fed to the alkylation step. The fraction (3) can also be re-fed to the alkylation step to undergo at least partial transalkylation, but preferably the transalkylation is effected in a specific reactor where this fraction of polyethylbenzenes is put in contact with benzene in the presence of a transalkylation catalyst.

A particular object of the present invention therefore relates to a process comprising the following steps:
(a) putting benzene in contact with ethanol, or a mixture of ethanol and ethylene, under gaseous or mixed gas-liquid phase conditions and in the presence of a catalytic system containing a zeolite belonging to the MTW group;
(b) subjecting the mixture resulting from step (a) to separation, to separate a fraction (1) containing benzene, a fraction (2) containing ethylbenzene and a fraction (3) containing polyethylbenzenes;
(c) putting the fraction (3) in contact with benzene, in the presence of a catalyst containing a zeolite, under transalkylation conditions, to obtain ethylbenzene.

The separation step can be carried out by using any of the known methods known to experts in the field. The alkylation product can be fractionated for example into a separation section using conventional separation methods, such as for example degassing, distillation and de-mixing of liquids.

The transalkylation reaction of step (c) can be carried out using any of the catalysts known to experts in the field for the transalkylation of polyethylbenzenes with benzene, in particular it can be conveniently effected in the presence of beta zeolite or Y zeolite or a catalyst based on zeolite beta or zeolite Y, in particular prepared according to what is described in EP 687500, EP 847802 and WO2004056475. In particular in WO2004056475 a catalyst is described comprising Y zeolite and an inorganic ligand, wherein the inorganic ligand is γ-alumina, characterized by a pore volume, obtained by summing the mesoporosity and macroporosity fraction present in the same catalyst, greater than or equal to 0.7 cc/g, wherein at least 30% of said volume consists of pores having a diameter greater than 100 nanometers.

The temperature conditions for the transalkylation reaction can be selected from 100°C to 350°C, the pressure is selected from 10 to 50 atm and the WHSV ranges from 0.1 hours⁻¹ to 200 hours⁻¹.

Reaction conditions which can be well-used are, for example, those described in EP 687500, EP 847802 and WO2004056475.

The transalkylation reaction product of step (c) is fractionated using the conventional separation methods, for example those described above for the separation step (b). In particular, a preferred aspect is to use the same separation section adopted for the separation step (b), feeding the mixture resulting from step (c) to said step (b).

A particular aspect of the present invention therefore relates to a process comprising the following steps:
(a) putting benzene in contact with ethanol, or a mixture of ethanol and ethylene, under gaseous or mixed gas-liquid phase conditions and in the presence of a catalytic system containing a zeolite belonging to the MTW group;
(b) subjecting the mixture resulting from step (a) to separation, to separate a fraction (1) containing benzene, a fraction (2) containing ethylbenzene and a fraction (3) containing polyethylbenzenes;
(c) putting the fraction (3) in contact with benzene, in the presence of a catalyst containing a zeolite, under transalkylation conditions;
(d) re-feeding the product resulting from step (c) to step (b);
(e) possibly re-feeding the fraction (1) resulting from step (b) to step (a) and/or step (c).

An objective of the present invention also relates to a process for preparing styrene which comprises the following steps:
(a) putting benzene in contact with ethanol, or a mixture of ethanol and ethylene, under gaseous or mixed gas-liquid phase conditions and in the presence of a catalytic system containing a zeolite belonging to the MTW group;
(b) subjecting the mixture resulting from step (a) to separation, to separate a fraction (1) containing benzene, a fraction (2) containing ethylbenzene and a fraction (3) containing polyethylbenzenes;
(c) possibly putting the fraction (3) in contact with benzene, in the presence of a catalyst containing a zeolite, under transalkylation conditions;
(d) possibly re-feeding the product resulting from step (c) to step (b);
(e) possibly re-feeding the fraction (1) resulting from step (b) to step (a) and/or step (c);
(f) subjecting the fraction (2) obtained in step (b) and containing ethylbenzene to dehydrogenation to obtain styrene.

Step (f) is well-known in literature and can be effected for example as described in US7,393,986.

The following examples are provided for illustrating the invention claimed herein, without however limiting its scope in any way.

### EXAMPLE 1

An alkylation test of benzene with ethyl alcohol is effected using the experimental device described hereunder.

The experimental device consists of tanks for the reagents benzene and ethyl alcohol, feeding pumps of the reagents to the reactor, a preheating unit of the reagents, a steel reactor situated inside an electric heating oven, a regulation loop of the temperature inside the reactor, a regulation loop of the pressure inside the reactor, a cooling agent of the reactor effluent and a collection system of the liquid and gaseous products.

In particular, the reactor consists of a cylindrical steel tube with a mechanical sealing system and diameter equal to about 2 cm.

Along the greater axis of the reactor, there is a thermometric cavity having a diameter equal to 1 mm inside which there is a thermocouple free to slide along the greater axis of the reactor.

A catalyst containing ZSM-12 zeolite, prepared as described in Example 2 of US 2003/0069459, is charged into the reactor.

A quantity of inert material is charged above and below the catalytic bed to complete the bed. The reagents benzene and ethanol are fed to the reactor - preheated and premixed in a suitable mixer - with an up-flow.

The reaction products are analyzed gaschromatographically. The reaction conditions at which the test is effected are the following:
Reaction temperature: 220°C
Reaction pressure: 6 bar
WHSV: 1 hours⁻¹
[Benzene]/[ethanol] in the feed: 5.53 moles/moles

These conditions cause the reagents to be in gaseous phase and the products partially in liquid phase. The attribution of the physical state of the reagent mixture is obtained by both comparison with the existing phase diagrams for the components and mixtures in question, and also via calculation, adopting the RKS state equation (Soave. G. Chem. Eng. Sci 27, 1197, (1972)).

The interaction parameters for this equation are obtained from the regression of the experimental data of literature relating to liquid-vapour equilibriums and reciprocal solubilities of the hydrocarbon-water mixtures (C.C. Li, J.J.McKetta Jul. Chem. Eng. Data 8 271-275 (1963) and C. Tsonopoulos, G.M. Wilson ALCHE Journe129,990-999, (1983)).

The reaction system to which the above equation is applied, is assimilated, with respect to the compositions, to the system
[benzene]/[ethylene] = 5.53 and
[benzene]/[water] = 5.53

The total concentration of water present in the complete conversion system of the reagent ethanol is equal to about 3.75%.

The selectivity during the whole duration of the test remained unaltered with values equal to about 70% for the [EB]/[ethanol] selectivity and about 92% for the [Ar]/[ethanol] selectivity.

## Claims

1. A process for the alkylation of benzene with ethanol, or a mixture of ethanol and ethylene, comprising effecting said alkylation reaction under gaseous phase or mixed gas-liquid phase conditions and in the presence of a catalytic system containing a zeolite belonging to the MTW family.

2. The process according to claim 1, wherein the pressure and temperature operating conditions correspond to complete gas phase of the whole mixture present in the reaction section.

3. The process according to claim 1, wherein the pressure and temperature operating conditions correspond to at least partial liquid phase of the reaction products.

4. The process according to claim 1, wherein the pressure and temperature operating conditions are such as to have reagents and products in both gas phase and liquid phase.

5. The process according to claim 1, wherein ZSM-12 zeolite is used as MTW zeolite.

6. The process according to claim 1, wherein the zeolite is used in the form in which the cationic sites present in the zeolite are occupied for at least 50% by hydrogen ions.

7. The process according to claim 1, wherein the zeolite is used in the form bound with a ligand.

8. The process according to claim 7, wherein the ligand is selected from alumina, silica, a silico-aluminate, titania, zirconia or clay.

9. The process according to one or more of the previous claims effected at a temperature ranging from 200 to 400°C and a pressure ranging from 1 to 20 bar.

10. The process according to claim 9, wherein the pressure is lower than 10 bar.

11. The process according to claim 10, wherein the pressure ranges from 1 to 6 bar.

12. The process for the alkylation of benzene according to claim 1, wherein the molar ratio between benzene and ethanol, or between benzene and the mixture of ethanol and ethylene, varies from 2 to 20.

13. The process for the alkylation of benzene according to the previous claim, wherein the reaction is carried out with a molar ratio between benzene and ethanol, or between benzene and the mixture of ethanol and ethylene, varies from 4 to 10.

14. The process according to claim 1, wherein, when the alkylating mixture consists of ethanol and ethylene, the reaction is carried out with a molar ratio between ethanol and ethylene ranging from 10 to 0.01.

15. The process for the alkylation of benzene according to the previous claim, wherein the reaction is carried out with a molar ratio between ethanol and ethylene ranging from 5 to 0.1.

16. The process according to one or more of the previous claims, wherein the catalytic system contains phosphorous.

17. The process according to claim 14, wherein the catalytic system contains phosphorous in a quantity of less than 3% by weight with respect to the total weight of the catalytic composition.

18. The process according to one or more of the previous claims, wherein the ethanol used is obtained by the transformation of biomasses.

19. The process according to claim 18, wherein the ethanol is obtained from biomasses by means of a process comprising a step in which the biomass is transformed into a feed which can be used for fermentation, and a step in which the feed thus obtained is subjected to fermentation.

20. The process according to claim 18, wherein the ethanol is obtained by means of a process in which the biomass is simultaneously transformed into a feed which can be used for fermentation, and said feed undergoes fermentation.

21. A process for preparing ethylbenzene comprising a process for the alkylation of benzene according to one or more of the previous claims, wherein the mixture resulting from alkylation is subjected in a step (b) to separation for separating a fraction (1) containing benzene, a fraction (2) containing ethylbenzene and a fraction (3) containing polyethylbenzenes, and wherein said fraction (3) is put in contact, in a step (c), with benzene, in the presence of a catalyst containing a zeolite, under transalkylation conditions, to obtain ethylbenzene.

22. The process according to claim 21, wherein the catalyst used in step (c) contains beta zeolite or Y zeolite.

23. The process according to claim 21, wherein the transalkylation reaction of step (c) is carried out at a temperature ranging from 100°C to 350°C, at a pressure ranging from 10 to 50 atm and a WHSV ranging from 0.1 hours⁻¹ to 200 hours⁻¹.

24. The process according to one or more of the previous claims 21-23, wherein the product resulting from step (c) is re-fed to step (b), and possibly the fraction (1) resulting from step (b) is re-fed to step (a) and/or step (c).

25. A process according to one or more of the previous claims, for preparing styrene, comprising the following steps:
(a) putting benzene in contact with ethanol, or a mixture of ethanol and ethylene, under gaseous or mixed gas-liquid phase conditions and in the presence of a catalytic system containing a zeolite belonging to the MTW group;
(b) subjecting the mixture resulting from step (a) to separation, to separate a fraction (1) containing benzene, a fraction (2) containing ethylbenzene and a fraction (3) containing polyethylbenzenes;
(c) putting the fraction (3) in contact with benzene, in the presence of a catalyst containing a zeolite, under transalkylation conditions;
(d) possibly re-feeding the product resulting from step (c) to step (b);
(e) possibly re-feeding the fraction (1) resulting from step (b) to step (a) and/or step (c);
(f) subjecting the fraction (2) obtained in step (b) and containing ethylbenzene to dehydrogenation to obtain styrene.

## Patentansprüche

1. Verfahren zur Alkylierung von Benzol mit Ethanol oder einer Mischung von Ethanol und Ethylen, das umfasst, dass die Alkylierungsreaktion unter Gasphasen- oder gemischten Gas-Flüssigphasenbedingungen und in der Gegenwart eines katalytischen Systems, das einen Zeolithen enthält, der zu der MTW-Familie gehört, durchgeführt wird.

2. Verfahren nach Anspruch 1, wobei sich die Druck- und Temperaturbetriebsbedingungen auf die ganze Gasphase der gesamten Mischung, die in dem Reaktionsabschnitt vorliegt, beziehen.

3. Verfahren nach Anspruch 1, wobei sich die Druck- und Temperaturbetriebsbedingungen auf eine zumindest teilweise flüssige Phase der Reaktionsprodukte beziehen.

4. Verfahren nach Anspruch 1, wobei die Druck- und Temperaturbetriebsbedingungen so sind, dass die Reagenzien und Produkte sowohl in der Gasphase als auch der flüssigen Phase vorliegen.

5. Verfahren nach Anspruch 1, wobei ZSM-12-Zeolith als MTW-Zeolith verwendet wird.

6. Verfahren nach Anspruch 1, wobei der Zeolith in einer Form verwendet wird, in welcher die in dem Zeolithen vorliegenden kationischen Stellen zu zumindest 50% durch Wasserstoffionen besetzt sind.

7. Verfahren nach Anspruch 1, wobei der Zeolith in einer mit einem Liganden gebundenen Form verwendet wird.

8. Verfahren nach Anspruch 7, wobei der Ligand aus Aluminiumoxid, Silica, einem Silicoaluminat, Titandioxid, Zirconiumdioxid oder Ton ausgewählt wird.

9. Verfahren nach einem oder mehreren der vorstehenden Ansprüche, das bei einer Temperatur, die von 200 bis 400 °C reicht, und einem Druck, der von 1 bis 20 bar reicht, durchgeführt wird.

10. Verfahren nach Anspruch 9, wobei der Druck niedriger als 10 bar ist.

11. Verfahren nach Anspuch 10, wobei der Druck von 1 bis 6 bar reicht.

12. Verfahren zur Alkylierung von Benzol nach Anspruch 1, wobei das Molverhältnis zwischen Benzol und Ethanol oder zwischen Benzol und der Mischung von Ethanol und Ethylen von 2 bis 20 variiert.

13. Verfahren zur Alkylierung von Benzol nach dem vorstehenden Anspruch, wobei die Reaktion mit einem Molverhältnis zwischen Benzol und Ethanol oder zwischen Benzol und der Mischung von Ethanol und Ethylen, das von 4 bis 10 variiert, durchgeführt wird.

14. Verfahren nach Anspruch 1, wobei, wenn die alkylierende Mischung aus Ethanol und Ethylen besteht, die Reaktion mit einem Molverhältnis zwischen Ethanol und Ethylen, das von 10 bis 0,01 reicht, ausgeführt wird.

15. Verfahren zur Alkylierung von Benzol nach dem vorstehenden Anspruch, wobei die Reaktion mit einem Molverhältnis zwischen Ethanol und Ethylen, das von 5 bis 0,1 reicht, ausgeführt wird.

16. Verfahren nach einem oder mehreren der vorstehenden Ansprüche, wobei das katalytische System Phosphor enthält.

17. Verfahren nach Anspruch 14, wobei das katalytische System Phosphor in einer Menge von weniger als 3 Gewichts-% in Bezug auf das Gesamtgewicht der katalytischen Zusammensetzung enthält.

18. Verfahren nach einem oder mehreren der vorstehenden Ansprüche, wobei das verwendete Ethanol durch die Umwandlung von Biomassen erhalten wird.

19. Verfahren nach Anspruch 18, wobei das Ethanol aus Biomassen durch ein Verfahren erhalten wird, das einen Schritt, in welchem die Biomasse in eine Beschickung, welche für eine Fermentierung verwendet werden kann, umgewandelt wird, und einen Schritt, in welchem die so erhaltene Beschickung einer Fermentierung unterzogen wird, umfasst.

20. Verfahren nach Anspruch 18, wobei das Ethanol durch ein Verfahren erhalten wird, in welchem die Biomasse gleichzeitig in eine Beschickung, welche für eine Fermentierung verwendet werden kann, umgewandelt wird und die besagte Beschickung einer Fermentierung ausgesetzt wird.

21. Verfahren zum Herstellen von Ethylbenzol umfassend ein Verfahren zur Alkylierung von Benzol nach einem oder mehreren der vorstehenden Ansprüche, wobei die Mischung, die sich aus der Alkylierung ergibt, in einem Schritt (b) dem Abtrennen einer Fraktion (1), die Benzol enthält, einer Fraktion (2), die Ethylbenzol enthält, und einer Fraktion (3), die Polyethylbenzole enthält, unterzogen wird und wobei die besagte Fraktion (3) in einem Schritt (c) mit Benzol in der Gegenwart eines Katalysators, der einen Zeolithen enthält, unter Transalkylierungsbedingungen in Kontakt gebracht wird, um Ethylbenzol zu erhalten.

22. Verfahren nach Anspruch 21, wobei der im Schritt (c) verwendete Katalysator Beta-Zeolith oder Y-Zeolith enthält.

23. Verfahren nach Anspruch 21, wobei die Transalkylierungsreaktion von Schritt (c) bei einer Temperatur, die von 100 °C bis 350 °C reicht, bei einem Druck, der von 10 bis 50 atm reicht, und einer WHSV, die von 0,1 Stunden⁻¹ bis 200 Stunden⁻¹ reicht, ausgeführt wird.

24. Verfahren nach einem oder mehreren der vorstehenden Ansprüche 21-23, wobei das sich aus Schritt (c) ergebende Produkt zu Schritt (b) rückgeführt wird und möglicherweise die sich aus Schritt (b) ergebende Fraktion (1) zu Schritt (a) und/oder Schritt (c) rückgeführt wird.

25. Verfahren nach einem oder mehreren der vorstehenden Ansprüche zum Herstellen von Styrol, das die folgenden Schritte umfasst:
(a) Inkontaktbringen von Benzol mit Ethanol oder einer Mischung von Ethanol und Ethylen unter Gasphasen- oder gemischten Gas-Flüssigphasenbedingungen und in der Gegenwart eines katalytischen Systems, das einen Zeolithen enthält, der zu der MTW-Gruppe gehört,
(b) Unterziehen der sich aus Schritt (a) ergebenden Mischung einer Trennung, um eine Fraktion (1), die Benzol enthält, eine Fraktion (2), die Ethylbenzol enthält, und eine Fraktion (3), die Polyethylbenzole enthält, abzutrennen,
(c) Inkontaktbringen der Fraktion (3) mit Benzol in der Gegenwart eines Katalysators, der einen Zeolithen enthält, unter Transalkylierungsbedingungen,
(d) möglicherweise Rückführen des sich aus Schritt (c) ergebenden Produkts zu Schritt (b),
(e) möglicherweise Rückführen der sich aus Schritt (b) ergebenden Fraktion (1) zu Schritt (a) und/oder Schritt (c),
(f) Unterziehen der Fraktion (2), die in Schritt (b) erhalten worden ist und Ethylbenzol enthält, einer Dehydrierung, um Styrol zu erhalten.

## Revendications

1. Procédé d'alkylation du benzène avec de l'éthanol, ou avec un mélange d'éthanol et d'éthylène, comprenant le fait d'effectuer cette réaction d'alkylation dans des conditions dans lesquelles il se forme une phase gazeuse ou un mélange de phases gazeuse et liquide, et en présence d'un système catalytique qui contient une zéolithe appartenant à la famille MTW.

2. Procédé conforme à la revendication 1, dans lequel les conditions opératoires de température et de pression sont telles que tout le mélange présent dans la zone de réaction forme une phase totalement gazeuse.

3. Procédé conforme à la revendication 1, dans lequel les conditions opératoires de température et de pression sont telles qu'au moins une partie des produits de réaction forment une phase liquide.

4. Procédé conforme à la revendication 1, dans lequel les conditions opératoires de température et de pression sont telles que réactifs et produits forment deux phases, une phase gazeuse et une phase liquide.

5. Procédé conforme à la revendication 1, dans lequel on utilise de la zéolithe ZSM-12 en tant que zéolithe MTW.

6. Procédé conforme à la revendication 1, dans lequel la zéolithe est utilisée sous une forme dans laquelle au moins 50 % des sites cationiques présents dans la zéolithe sont occupés par des ions hydrogène.

7. Procédé conforme à la revendication 1, dans lequel la zéolithe est utilisée sous une forme liée à un ligand.

8. Procédé conforme à la revendication 7, dans lequel le ligand est choisi parmi de l'alumine, de la silice, un silico-aluminate, de l'oxyde de titane, de la zircone et une argile.

9. Procédé conforme à l'une ou plusieurs des revendications précédentes, qui est mis en oeuvre à une température qui vaut de 200 à 400 °C et sous une pression qui vaut de 1 à 20 bars.

10. Procédé conforme à la revendication 9, dans lequel la pression est inférieure à 10 bars.

11. Procédé conforme à la revendication 10, dans lequel la pression vaut de 1 à 6 bars.

12. Procédé d'alkylation du benzène, conforme à la revendication 1, dans lequel le rapport molaire entre le benzène et l'éthanol, ou entre le benzène et le mélange d'éthanol et d'éthylène, vaut de 2 à 20.

13. Procédé d'alkylation du benzène, conforme à la revendication précédente, dans lequel on met en oeuvre la réaction avec un rapport molaire entre le benzène et l'éthanol, ou entre le benzène et le mélange d'éthanol et d'éthylène, qui vaut de 4 à 10.

14. Procédé conforme à la revendication 1, dans lequel, si le mélange alkylant est constitué d'éthanol et d'éthylène, on met en oeuvre la réaction avec un rapport molaire entre l'éthanol et l'éthylène valant de 10 à 0,01.

15. Procédé d'alkylation du benzène, conforme à la revendication précédente, dans lequel on met en oeuvre la réaction avec un rapport molaire entre l'éthanol et l'éthylène valant de 5 à 0,1.

16. Procédé conforme à l'une ou plusieurs des revendications précédentes, dans lequel le système catalytique contient du phosphore.

17. Procédé conforme à la revendication 14, dans lequel le système catalytique contient du phosphore en une quantité qui représente moins de 3 % du poids total de la composition catalytique.

18. Procédé conforme à l'une ou plusieurs des revendications précédentes, pour lequel l'éthanol utilisé a été obtenu par transformation de biomasses.

19. Procédé conforme à la revendication 18, pour lequel on a obtenu l'éthanol à partir d'une biomasse, selon un procédé comprenant une étape dans laquelle la biomasse est transformée en une matière d'alimentation qui peut être utilisée pour une fermentation, et une étape dans laquelle la matière d'alimentation ainsi obtenue subit une fermentation.

20. Procédé conforme à la revendication 18, pour lequel on a obtenu l'éthanol selon un procédé dans lequel, simultanément, la biomasse est transformée en une matière d'alimentation qui peut être utilisée pour une fermentation et cette matière d'alimentation subit une fermentation.

21. Procédé de préparation d'éthyl-benzène, comprenant un procédé d'alkylation du benzène conforme à l'une ou plusieurs des revendications précédentes, dans lequel, au cours d'une étape (b), on soumet le mélange résultant de l'alkylation à une opération de séparation, afin de séparer
1) une fraction contenant du benzène,
2) une fraction contenant de l'éthyl-benzène,
3) et une fraction contenant des polyéthyl-benzènes,
et dans lequel, au cours d'une étape (c), on met cette fraction (3) en contact avec du benzène, en présence d'un catalyseur contenant une zéolithe, dans des conditions appropriées pour une trans-alkylation, afin d'obtenir de l'éthyl-benzène.

22. Procédé conforme à la revendication 21, dans lequel le catalyseur utilisé dans l'étape (c) contient une zéolithe bêta ou une zéolithe Y.

23. Procédé conforme à la revendication 21, dans lequel la réaction de trans-alkylation de l'étape (c) est mise en oeuvre à une température qui vaut de 100 à 350 °C, sous une pression qui vaut de 10 à 50 atm, et avec une vitesse spatiale horaire en poids (WHSV) qui vaut de 0,1 à 200 h⁻¹.

24. Procédé conforme à l'une ou plusieurs des revendications 21 à 23 précédentes, dans lequel le produit issu de l'étape (c) est renvoyé vers l'étape (b), et la fraction (1) issue de l'étape (b) est éventuellement renvoyée vers l'étape (a) et/ou l'étape (c).

25. Procédé de préparation de styrène, conforme à l'une ou plusieurs des revendications précédentes, lequel procédé comporte les étapes suivantes :
a) mettre du benzène en contact avec de l'éthanol ou avec un mélange d'éthanol et d'éthylène, dans des conditions dans lesquelles il se forme une phase gazeuse ou un mélange de phases gazeuse et liquide, et en présence d'un système catalytique qui contient une zéolithe appartenant à la famille MTW ;
b) soumettre le mélange issu de l'étape (a) à une opération de séparation, afin de séparer
1) une fraction contenant du benzène,
2) une fraction contenant de l'éthyl-benzène,
3) et une fraction contenant des polyéthyl-benzènes ;
c) mettre la fraction (3) en contact avec du benzène, en présence d'un catalyseur contenant une zéolithe, dans des conditions appropriées pour une trans-alkylation ;
d) éventuellement, renvoyer vers l'étape (b) le produit résultant de l'étape (c) ;
e) éventuellement, renvoyer vers l'étape (a) et/ou l'étape (c) la fraction (1) résultant de l'étape (b) ;
f) et soumettre à une déshydrogénation la fraction (2) obtenue dans l'étape (b), qui contient de l'éthyl-benzène, pour obtenir du styrène.
